# EUROPEAN PATENT APPLICATION

(11) **EP 3 599 284 A1**
(43) Date of publication of application: **29.01.2020**
(21) Application number: 18185987.7
(22) Date of filing: 27.07.2018
(51) Int. Cl.: C12Q 1/6869

(54) **SIMULTANEOUS DETECTION OF MULTIPLE NUCLEIC ACID TEMPLATES USING MODIFIED PRIMERS**

(71) Applicant: Bio-Id Diagnostic Inc., Edmonton, Alberta T5S 1E6 (CA)
(72) Inventor: Vinayagamoorthy, Thuraiayah, Edmonton, Alberta T5S 1E6 (CA)
(74) Representative: V.O.

(57) **Abstract**

The invention involves detection of multiple nucleic acid polymer templates. These templates include genetic variants such as deletion, insertion, fusion, gene expression or detection of specific genomes in a sample with heterogeneous genomes. The process includes amplification of a specific segment of genetic material for example by polymerase chain reaction or ligase chain reaction, followed by DNA and/or RNA sequencing. The invention is based on the modified primers used for amplification that comprise assay-specific nucleotide sequences, and positioning of the sequencing primer in the amplicon. Based on the nucleotide sequence generated, the genetic variants and/or specific genomes are determined.

## Description

The invention refers to a method of detecting one or more deoxyribose nucleotide (DNA) template(s) by amplifying segments of the DNA template by polymerase chain reaction using upper and lower primers, generating a nucleotide sequence and aligning the generated nucleotide sequence with a reference nucleotide sequence to determine the location of an indicator nucleotide in the reference nucleotide sequence and the DNA template, and to identify the DNA template. In addition, the invention is directed to a kit for detecting one or more DNA templates.

### FIELD OF INVENTION

All forms of life, including humans, animals and microbes, carry a species-specific genome comprising a nucleic acid polymer, made up of either DNA or RNA. Further, these genomes (DNA and RNA) undergo changes that include addition or deletion of one or more nucleotides and/or gene fusion and nucleotide substitution. Some of these variations are associated with known disease conditions, hence their detection is critical for diagnosis, prognosis and treatment, respectively. The embodiments of this invention include, but are not limited to, human and animal disease conditions, respectively, DNA fingerprinting for forensic applications, paternity testing, and detection of microbial speciation.

### BACKGROUND INFORMATION

With the elucidation of the structure of DNA and the completion of the human genome project, studies were accelerated to identify genetic changes that are correlated with disease conditions. Therefore, there is need to detect these genetic changes, for diagnosis, prognosis, treatment and overall management of patients. Over the past decades, there have been numerous medical and scientific reports claiming association of genetic variations with specific disease conditions (1). These genetic variations could be either germline mutations (hereditary), or somatic mutation (acquired). Both germline and somatic mutations include nucleotide substitutions, deletions or additions of one or more nucleotides. The deletions and/or the insertions comprise from one nucleotide to several nucleotides. Further, the same genetic changes are found in either one allele (loss of heterozygosity) or both alleles (homozygosity).

DNA sequencing is the most accurate procedure to detect genetic variation. Presently, there are several sequencing platforms, including Sanger sequencing, pyrosequencing, next generation sequencing, sequencing by nanopore and RNA sequencing. Sanger sequencing is considered a reference and confirmatory method for sequencing a DNA or an RNA template. Although all sequencing platforms are applicable, this invention is illustrated for example by using Sanger sequencing.

### SUMMARY OF INVENTION

The instant invention refers to a method of detecting one or more deoxyribose nucleotide (DNA) template(s) comprising the steps of:
- amplifying segments of a DNA template by polymerase chain reaction using upper primers and lower primers, wherein each of the lower primers comprises a homopolymer region at its 5' end comprising a homopolymer detection and an indicator region, and repeated nucleotides, wherein the number of repeated nucleotides in the lower primers are identical or differ from each other, and
- generating a nucleotide sequence consisting of a nucleotide sequence that is complimentary to the lower primer, the detection region and the indicator region using a sequencing primer annealing within an amplicon, and
- aligning the generated nucleotide sequence with a reference sequence to determine the location of the indicator nucleotide in the reference nucleotide sequence and in the DNA template and thereby identifying the DNA template.

In the instant invention, the DNA template optionally comprises a genetic variant comprising a deletion region compared to the corresponding wild type DNA template, and the upper primers and lower primers amplify a segment of the DNA template encompassing the deletion, where the upper primers anneal upstream of the deletion region and the lower primers anneal downstream of the deletion region, and the sequencing primer anneals within the amplicon upstream of the deletion generating a nucleotide sequence that corresponds to a nucleotide sequence downstream of the deletion region, and the generated nucleotide sequence is complimentary to the lower primer, the homopolymer detection region and the indicator region at the 5' end.

Alternatively, the DNA template comprises a genetic variant comprising an insertion region compared to the corresponding wild type DNA template, and the upper primers and the lower primers amplify a segment of the DNA template encompassing the insertion region, where the upper primers anneal upstream of the insertion region and the lower primers anneal downstream of insertion region, and the sequencing primer anneals within the amplicon upstream of the insertion region generating a nucleotide sequence that corresponds to a nucleotide sequence down-stream of the insertion region, and the generated nucleotide sequence is complimentary to the lower primer, the homopolymer detection region and the indicator region at the 5' end.

Alternatively, the DNA templates comprises a genetic variant comprising a substitution of one or more nucleotide sequences of deoxyribose nucleotide.

In the instant invention, the upper primer for example comprises a homopolymer region at its 5' end comprising a detection and an indicator region and optionally, both the upper primer and the lower primer comprise the homopolymer region at the 5' end comprising a detection and an indicator region.

Amongst others, the DNA template comprises a ribose nucleotide and in some embodiments the amplification is optionally performed by ligase chain reaction.

According to an embodiment of the invention a part of the upper primer anneals upstream of the deletion region and the other part of the upper primer anneals downstream of the deletion region. A part of a primer in any context of the instant invention comprises for example 2 to 12 nucleotides, e.g., 2, 3, 4, 5, 6, 7, 8, 9, 10, 11 or 12 nucleotides. An example of a primer annealing around a deletion region is the following:
EGFR Del 746-A750

| | |
|---|---|
| Upper primer | TATCAA................................AA-3' |
| | TATCAA*GGAATTAAGAGAAGC*AACATC |

In the instant invention a part of the sequencing primer for example anneals upstream of the deletion region and the other part of the upper primer anneals downstream of the deletion region, and/or a part of the upper primer anneals in the insertion region and the other part anneals downstream of the insertion region, or the upper primer anneals completely in the insertion region, and/or a part of the sequencing primer anneals in the insertion region and the other part anneals downstream of the insertion region, and/or the sequencing primer anneals completely in the insertion region.

The instant invention includes a method comprising at least two sequencing primers, one binding specifically to a mutated allele comprising a mutant nucleotide at its 3' end and annealing within the region of the amplicon generating a nucleotide sequence that consists of a nucleotide sequence downstream of a nucleotide substitution locus, wherein the nucleotide sequence is complimentary to the lower primer, and the detection and indicator region, and that the other sequencing primer is specifically binding the wild type allele comprising the wildtype nucleotide at its 3' end, annealing within the region of the amplicon generating a nucleotide sequence that consists of a nucleotide sequence downstream of the nucleotide substitution locus, wherein the nucleotide sequence is complimentary to the lower primer, and the detection and indicator region and, wherein the generated nucleotide sequence is aligned with the nucleotide sequence of the wild type, the difference of the location of the marker nucleotide between the wild type nucleotide sequence and the generated nucleotide sequence determines the presence of the deletion in the DNA template.

In addition, the invention includes a method, wherein a fusion DNA template comprises an insertion region, and the upper primers and the lower primers amplify a segment of the DNA template encompassing the fusion region, where the upper primers anneal upstream of the region junction of the fusion and the lower primers anneal down- stream of the junction of the fusion region, and the sequencing primer (one or more) anneals within the amplicon upstream of the junction of the fusion region generating a nucleotide sequence that corresponds to a nucleotide sequence down-stream of the junction of the fusion region, and the generated nucleotide sequence is complimentary to the lower primer, the homopolymer detection region and the indicator region at the 5' end.

Moreover, the invention includes a method, wherein the DNA template comprises different genomes, and the upper primers and lower primers amplify a segment of the DNA template, the genome specific sequencing primer anneals within the amplicon generating a nucleotide sequence complimentary to the lower primer, the homopolymer detection region and the indicator region at the 5' end and the location of the indicator region identifies its corresponding genome.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### BRIEF DESCRIPTION OF THE DRAWINGS

Figure 1 illustrates various components of the invention: (a) Shows the primer annealing region, detection region and the indicator region of one of the primers such as PCR primers and b) shows the location of the primers such as PCR primers, sequencing primers and the region of genetic variants.
Figure 2 illustrates (a) homologous deletion and (b) homologous insertion.
Figure 3 illustrates (a) loss of heterozygosity (Deletion) and (b) gain of heterozygosity (Insertion).
Figure 4 shows (a) position of the sequencing primers with one allele having a deletion and (b) position of the sequencing primers with one allele having an insertion.
Figure 5 shows (a) positions of the sequencing primer(s) with both alleles having a substitution and (b) positions of the sequencing primer(s) with one allele having a substitution.
Figure 6 depicts an electropherogram showing wildtype alleles with homopolymer region and the indicator region.
Figure 7 depicts an electropherogram showing a wildtype allele and an allele with deletion where the positioning of the indicator is shown.

### DETAILED DESCRIPTION OF THE INVENTION

The invention involves detection of multiple nucleic acid polymer templates. These templates include genetic variants such as deletion, insertion, fusion, gene expression or detection of specific genomes in a sample with heterogeneous genomes. The process includes amplification of a specific segment of genetic material by polymerase chain reaction or ligase chain reaction, followed by DNA and/or RNA sequencing. The invention is based on the modified primers used for amplification that carry specific nucleotide sequences, and positioning of the sequencing primer in the amplicon. Based on the nucleotide sequence generated, the genetic variants and/or specific genomes are determined.

Deletion, insertion, fusion and substitutions are genetic aberrations that occur in genomes of all life forms. Deletion is where a stretch of the DNA or RNA is deleted for example during replication, insertion is where a stretch of DNA or RNA is added to the genome for example during replication. Fusion is where DNA or RNA stretches from two or more genes come together, called gene fusion. Fusion occurs for example during transcription known as fusion transcript. Substitution is where a (one or more) nucleotide is replaced by another one (one or more) for example during replication. If more than one nucleotide is replaced the replaced nucleotides are directly next to each other or separated by one or more non-replaced nucleotide(s).

A typical molecular method includes three basic steps: total nucleic acid extraction (DNA, RNA), amplification of specific segments of the extracted nucleic acid using an amplification step such as polymerase chain reaction, followed by an identification step. The extraction is a very basic process, where the cells carrying the genetic materials are broken enzymatically and the nucleic acid is purified using magnetic beads. Extraction is followed by an amplification step where a specific segment of the DNA or RNA is amplified for example by polymerase chain reaction or ligase chain reaction using target specific primers. Once the amplification is completed the amplified products (i.e., amplicons) are identified by various methods including for example end-point PCR (Fragment analysis), real time PCR, micro array analysis, mass spectrometry, or DNA sequencing. Two of the most common technologies presently used in detecting allele specific nucleotides are for example Southern blotting using radio isotopes, and/or fragment analysis using fluorophores. Since these identification methods are based on the size of the amplicons generated, it is not reliably verifiable and may lead to erroneous conclusion. There is therefore a need to improve identification of the above mentioned genetic variations. Sanger DNA sequencing is considered the "Gold Standard" for identification and confirmation of the intended target. Use of traditional Sanger sequencing is for example designed to sequence a single homogenous DNA template.

The method of this invention involves total DNA and/or RNA extraction, and amplification using for example polymerase chain reaction or ligase chain reaction. The amplicons are then sequenced using for example Sanger sequencing and the nucleotide sequence is for example determined by performing an electropherogram. Although this is a standard procedure, with two or more genetic materials (templates) in the same sample, for example one of the genetic templates comprising a deletion, the nucleotide sequence generated by the electropherogram may comprise overlapping (mixed) base calls, hence no valid nucleotide sequence may be derived. The reason is that DNA sequencing is so far the standard method of determining the nucleotide sequence of a DNA and/or an RNA template. Standard nucleic acid sequencing for example consists of a sequencing primer that anneals to one of the strands of the DNA template, and DNA polymerase randomly incorporates both dideoxynucleosides and deoxynucleotides, generating a series of truncated, single stranded DNA with the nucleotide at the 3' end comprising one of the four fluorophores attached to the dideoxynucleosides. When these labeled truncated molecules are separated for example through capillary electrophoresis, they pass for example the CCD camera and the fluorophore signals that they comprise are then for example recorded and analyzed to indicate the order of the nucleotide sequence of the template. For example, if the sample comprises only one type of DNA template (e.g. homozygous template), with sequencing primer having the same annealing sites on both of the alleles, then the nucleotide sequence generated is the same and readable (Table 2). If the sample is from a heterozygous template of two different alleles, one comprising a deletion, using the same sequencing primer, both the nucleotide sequences will overlap, resulting in mixed calls where no meaningful nucleotide sequence is derived. Similarly, if the sample is from a heterozygous template of two different alleles, one with an insertion using the same sequencing primer, both the nucleotide sequences overlap, resulting in mixed calls where no meaningful nucleotide sequence is derived

This invention provides a surprising modification to solve the above dilemma. Using a surrogate site, variations in a heterozygous template are detected. This invention is based on the modification of one or more for example two primers used in the amplification step (e.g. polymerase chain reaction or ligase chain reaction or ligase polymerization reaction) and the sequencing primer(s).

The method of the invention includes nucleic acid extraction, which is DNA extraction, RNA extraction or both, using standard procedures. There are several commercially available kits to perform such extraction. Total nucleic acid extraction is followed by amplification where more copies of the target DNA templates are generated. Such amplification is carried out by a method of amplification such as polymerase chain reaction or ligase chain reaction. Further, the amplification is symmetrical, for example using two primers an upper primer and a lower primer, or asymmetrical for example using one primer.

In the following, the elements of the instant invention will be described in more detail. These elements are listed with specific embodiments, however, it should be understood that they may be combined in any manner and in any number to create additional embodiments. The variously described examples and embodiments should not be construed to limit the instant invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the instant application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

The term "about" means a quantity, level, value, number, frequency, percentage, dimension, size, amount weight, length etc. that varies by as much as 10, 9, 8, 7, 6, 5, 4, 3, 2, 1 %from a reference quantity, level, value, number, frequency, percentage, dimension, size, amount weight, length etc..

The scientific basis of this invention is represented by the modification of one or more, e.g., one or two of the primer(s) used in the amplification step. The modified primer(s) encompass for example the deletion and/or the insertion region of the DNA template which are for example a genome or a clone. The modified primer(s) used for amplification have a homologous repetitive sequence at the 5' end, which represents the detection region. The repetitive nucleotide sequence is made up of either a single nucleotide or multiple nucleotides. Further, the detection region is modified based on the genetic variants. For example, if the genetic variants are double nucleotide repeats and/or insertions, then the detection region is of two nucleotide repeats, either the same or different pair to that of the genetic variant region. The number of repeat units in the detection region will be more than that of the genetic variants. For example, if the maximum deletion is 10 nucleotides, then the repeat detection region will have more than 10 nucleotides. The number of nucleotides in the homologous repetitive region is determined by the maximum number of units of potential deletion/addition of the allele. For example, Huntington's disease carries a three-nucleotide repeat, hence a homologous repetitive segment with a three-nucleotide repeat.

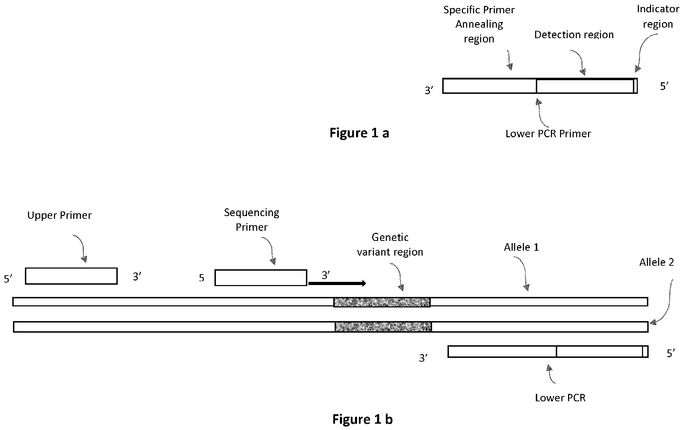

The repeats of the nucleotide are for example independent of the condition in the number of 1 to 100, 2 to 95, 3 to 85, 4 to 80, 5 to 75, 6 to 70, 7 to 65, 8 to 60, 9 to 55, 10 to 50, 11 to 45, 12 to 40, 13 to 35, 14 to 30, or 15 to 25. The number of the repeats is for example 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, or 95. At the distal end of the repetitive region there is an indicator region that will carry one or more nucleotides different from that of the homologous repetitive region (Fig. 1a, Table 2), wherein for example 1, 2, 3, 4, 5, 6, 7, 8, 9, 10, 11, 12, 13, 14, 15, 20 or 25 nucleotides are different.

The indicator region is/comprises the last 1 or 2 nucleotide(s) at the end of the detection region which differ from rest of the nucleotide(s) of the detection homopolymer region.

Following the amplification, the amplicons are sequenced using a target specific sequencing primer that sequences from the starting point through the modified primer such as a PCR primer to the end of the indicator region. The nucleotide sequence generated has two segments: the first segment is from the 3' end of the sequencing primer to the beginning of the homopolymer detection region and the second segment is the homopolymer region including the indicator region (Fig. 1b; Table 2). If there is no deletion or insertion of alleles, then the amplification will generate for example two identical amplicons. The amplicons generated are sequenced simultaneously, generating for example two nucleotide sequences; one from each of the two alleles. The nucleotide sequences generated from these two amplicons will be identical and the electropherogram will not have any mixed base reads. However, to rule out any homologous deletion or insertion, the read length of the nucleotide sequence is compared to the original wild-type. If the test read length is the same as that of the wild-type, then there is no deletion or insertion.

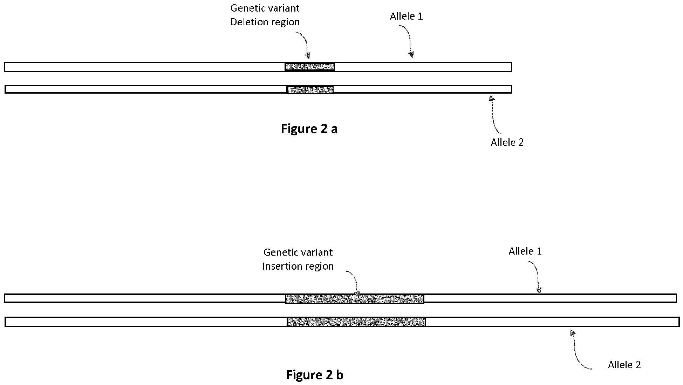

However, if the test read length is shorter than that of the wild-type, then there is a deletion in both alleles (Fig. 2a) and if the test read length is longer than the wild-type then there is an insertion in both alleles (Fig. 2b). The extent of the deletion is determined by the difference in the nucleotide read divided by the units of the variation. For example, if the difference in the nucleotide read between the wild-type and the test is 12 nucleotides and the unit of repeat is 3 nucleotides, then the genetic variation is 4 units. Similarly, the extent of the insertion is determined by the difference in the nucleotide read between the test and the wild-type divided by the units of the variation (Table 3). For example, if the difference in the nucleotide read is 20 nucleotides and the unit of repeat is 4 nucleotides, then the genetic variation is 5 units.

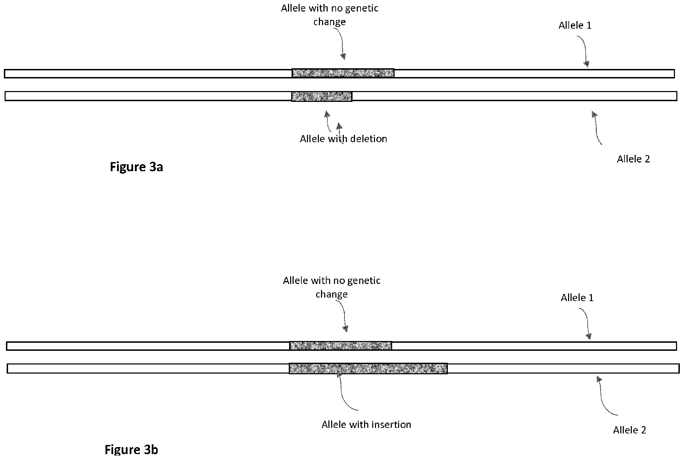

If there is a deletion in one of the alleles, the nucleotide sequence generated from the allele with the deletion will be shorter than the nucleotide sequence from the allele without any deletion (Fig. 3a). Similarly, if there is an insertion in one of the alleles, then the nucleotide sequence generated from the allele with the additional nucleotides is longer than that of the nucleotide sequences generated from the allele that did not have the additional nucleotides in the genome (Fig. 3b). In the case of either a deletion or an insertion, the nucleotide sequence has mixed base signals and be non-readable, except the nucleotide in the detection segment. For example, if there is one unit (three bases) deletion (i.e. segment between 3' end of the sequencing primer to the 5' end of the modified primer such as an PCR primer) in one of the alleles, there is a shift of three nucleotides to the left of the electropherogram, which in turn moves the indicator nucleotides three places to left (Table 2).

Similarly, if the genetic variation has an insertion of one unit (three bases) in one of the alleles, then there will be a shift of three nucleotides to the right of the electropherogram that in turn will move the indicator nucleotides three places to the right. The position of the indicator nucleotides among the two alleles confirms whether there is a deletion or addition. Hence the modification of the primer(s) such as a PCR primer is used as a surrogate region to detect the deletion or insertion in the DNA template.

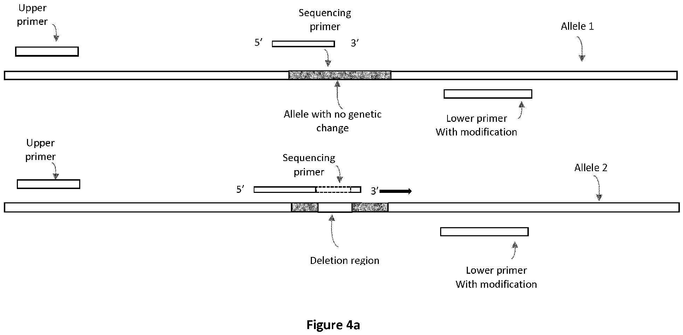

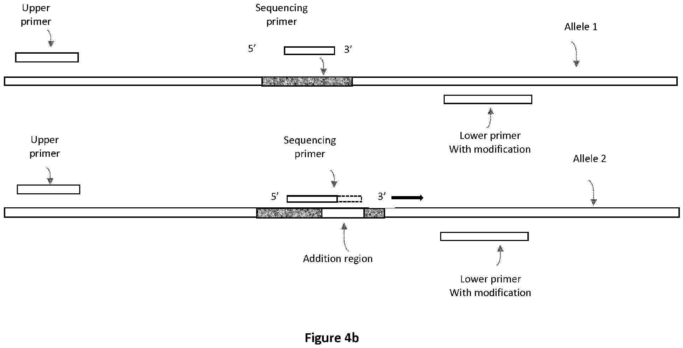

If there is a heterogeneous cell population where one or both of the allele(s) from one clonal cell at lower copy number comprise a deletion or an insertion, then the method uses a modified sequencing primer that anneals only to the allele carrying the deletion (Fig. 4a) or insertion (Fig. 4b). In the case of a deletion, the sequencing primer is designed in a way that the last few nucleotides at the 3' end, for example 1, 2, 3, 4, 5, 6, 7, 8, 9, or 10 nucleotides, follow immediately after the deletion region that the entire sequencing primer is constructed so that it binds to the deleted template and not to the wild-type. Similarly, if there is an insertion, the sequencing primer is designed so that the entire nucleotide sequence is from the inserted region that the entire sequencing primer binds to the inserted template and not to the wild-type. In case the inserted region is shorter (for example about 5 nucleotides), then most of the sequence will be located at the 5' end, and the 3' end comprises the sequence(s) from the inserted region.

The instant invention is for example used in the identification of prokaryotic genomes and the method comprises optionally an additional modification. The number of nucleotides from the 3' end of the sequencing primer to the indicator is genome specific. For example, for organism A, the number of nucleotides from the 3' end of the sequencing primer to the indicator is 28. For organism B the number of nucleotides from the 3' end of the sequencing primer to the indicator is for example 26 (Table 1). The method includes having a plasmid construct with an insert DNA that will have a baseline construct with annealing sites such as PCR annealing sites where one of the primers is modified as described above with an extended detection region and an indicator region, thus forming a reference nucleotide sequence. This construct also functions as an internal control. Similarly, for example organism A and organism B have genome specific annealing sites where one of the primers is modified as described above with a detection region and an indicator region. If both of the genomes are present, they are detected by the presence of a respective indicator region at the specified locus that overlaps with the signal in the detection region of the reference.

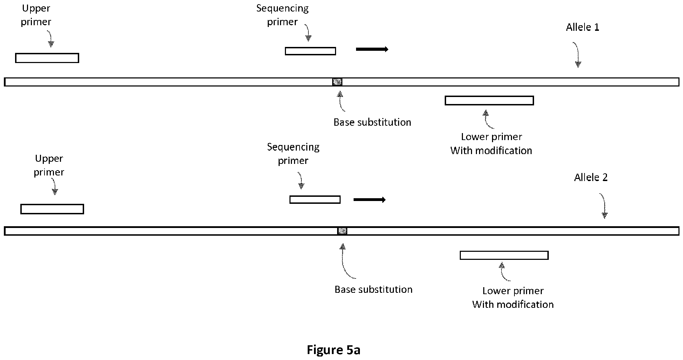

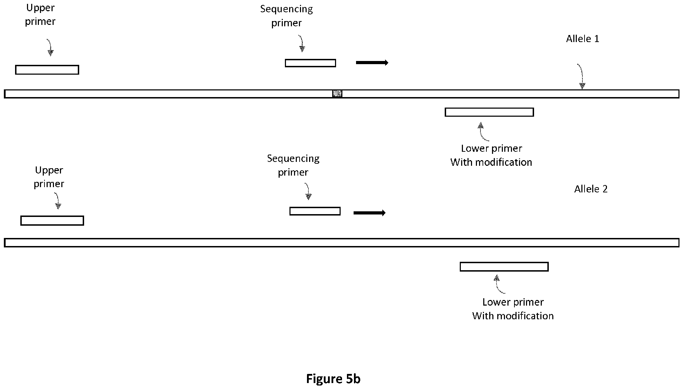

Moreover, the instant invention is for example used in the identification of a single base substitution. The method is the same as described above, but the sequencing step for example is modified. The sequencing step uses an allele specific sequencing primer at the 3' end (Fig. 5a and Fig. 5b). The sequencing primer for the mutant allele comprises the mutant nucleotide at its 3' end and the wild-type comprises the wild type nucleotide at its 3' end. The molecular weight of one of the sequencing primers is greater than the other. Hence, the nucleotide sequence generated from the one with higher molecular weight has its indicator appearing farther to the right than the one with lower molecular weight sequencing primer, hence both are detected.

The instant invention also refers for example to the determination of micro satellite variants that are associated with human diseases. Hereditary nonpolyposis colorectal cancer (HNPCC) commonly referred as Lynch syndrome is an autosomal hereditary condition that is associated with a number of cancers, including colorectal cancer. Lynch syndrome genetic changes occur for example in *MLH1, MSH2, MSH6,* or *PMS2* or an *EPCAM* gene that leads to defects in the DNA repair mechanism during cell replication. Genetic changes in MLH*1*, *MSH2, MSH6,* or *PMS2* or an *EPCAM* gene for example are direct evidence of Lynch syndrome. There is more than one mutation, some of which may not lead to defective repair. Hence, determination of defective repair is determined by its function. There are stretches of human DNA with single nucleotide repeats BAT 25, BAT 26 and dinucleotide repeats D2S123, D5S346, D17S250. If there are genetic defects due to mutations among MLH1, MSH2, MSH6, or PMS2 or an EPCAM genes that will be reflected in the number of repeats among repeats BAT 25, BAT 26 and dinucleotide repeats D2S123, D5S346, D17S250. The analysis and interpretation of these samples for MSI are challenging. (2,3).

Further, the invention is directed to the method for the detection of a trinucleotide variation found in Huntington's disease (4). There the trinucleotide cytosine-adenine-guanine (CAG) repeats in the Huntingtin gene exceeds about 39, which is considered a potential disease state and the individual will be affected. Early childhood detection of Huntington's disease could help in facilitating implementation of medical programs.

Another disease where the method of the instant invention is applicable for detection is cystic fibrosis for example, where more than three nucleotide deletions are involved (5). Cystic fibrosis is an autosomal germline mutation in both alleles of the human genome under disease conditions, and single allele mutation in carriers. The gene expresses cystic fibrosis transmembrane conductance regulator (CFTR) protein. The common mutation of the CFTR gene is for example three nucleotide deletion ΔF508 that results in the loss of amino acid phenylalanine at the 508 amino acid position. Traditionally cystic fibrosis is diagnosed measuring blood levels of immunoreactive trypsinogen. Presently cystic fibrosis diagnosis is carried out by molecular tests. This invention will be able to detect both single allele deletion and double allele deletions.

Another example for use of the method of the instant invention is detection of polymorphism in cytochrome oxidase genes associated with drug metabolism (6). The drug dosage is for example adjusted based on whether the patient is a slow or fast metabolizer. Cytochrome 2D6.9 2615_2617delAAG, 2C9*6 818 del A, are examples of deletions and *CYP3A4*6* 17661_176622 ins A and *CYP3A4*20* 25889_25890 ins A are examples of insertions.

Further, the instant invention refers to the detection of splice variants. More than 50% of gene expression undergoes variations (splice variants) in their expression; some of the transcripts may lose one or more exons (7,8). Such expression may result either in adding a few nucleotides at the exon:exon junction or in losing a few nucleotides at the exon:exon junction. These variations in the transcripts may result in proteins that may not have the epitope for their functionality, or if present, may not be accessible (7).

In an embodiment of the invention the method relates to DNA finger printing. There are several loci in the human genome with repetitive nucleotide sequences known for example as short tandem repeats (STR). These STRs are for example segregated independently from each other, and are used for example as markers to characterize the human genome (8). Further, a number of such nucleotide repeats for example vary among the two alleles in the same individual. Analysis of STR has become a standard procedure in DNA finger-printing. Application of DNA finger-printing include for example forensic science and paternity testing.

Moreover, the instant invention refers to the detection of genetic variations related to cancer. Apart from germline mutations, there are for example somatic mutations that occur during one's lifetime. Most of these mutations are associated with incidence and progression of cancer. This includes for example Epidermal growth factor receptor (EGFR) exon 19 in non-small cell lung cancer, which is detected for targeted therapy (9). It is also noted that in each of these cases there may be multiple variants (e.g. deletion in EGFR exon 19 may be 9 nucleotides, 15 nucleotides or any other number of nucleotides such as 10,11, 12, 13, 14, 16, 17, 18, 19, 20, 21, 22, 23, 24, 25, 26, 27, 28, 29, 30, 31, 32, 33, 34, 35, 36, 37, 38, 39, 40 etc.. Further, deletions are located anywhere in Exon 19. Other examples for genetic variations are in HER 2 exon 20 and/or MET exon 14, which together form a group of driver mutations in cancer (10,11). The instant invention further includes the detection of gene fusion for example Alk, ROS1, RET and NTRK (12,13,14,15).

Another embodiment of the invention is the detection of single nucleotide variant where a single nucleotide is substituted. This substitution is for example in human germline or a somatic mutation (Acquired). An example of germline mutations includes mutation G20210A (Prothrombin) and factor V Leiden that increases chance for blood clotting. Examples of somatic mutations (acquired) include for example Braf p.V600E a tyrosinase kinase mutation tested in late stage melanoma for treatment with vemurafenib, and Epidermal growth factor receptor mutation (EGFR L858R).

Moreover, the invention refers to a method for the detection of related microbial species. Microbes are part and parcel of human health, some of which cause specific diseases. Infectious diseases that are common among human and animals are for example caused by viruses, bacteria, fungi, and protozoa. Further, such infections are for example caused by multiple subspecies or subtypes that may cause the same disease. For example, vaginosis is caused by *Candida, albicans, Candida glabrata, Candida krusei,* and *Candida tropicalis* (16, Table 1). All are of clinical significance to adopt appropriate antifungal therapy for patient management. Another example would be human papillomavirus (HPV), which cause cancer of the cervix. There are a number of subtypes of HPV that cause cancer of the cervix (17). This invention detects for example subtypes of human papilloma virus causing cancer of the cervix or Candida species causing vaginosis in women.

The invention comprises any number of primers, i.e., upper, lower and sequencing primer, in the method to detect one or more templates and/or different positions of a template.

The method of the instant invention is used in assays to detect one or more DNA and/or RNA templates and the instant invention comprises such assays.

The instant invention is further directed to a kit for performing a method according to this invention, wherein the kit comprises one or more upper and/or lower primer(s), a sequencing primer, and optionally a reference sequence for detecting a DNA and/or RNA template.

### Examples

The following examples illustrate different embodiments of the present invention, but the invention is not limited to these examples.

### Example 1: Detection of Epidermal growth factor receptor. (e.g. deletion in EGFR exon 19)

There are four targeted chemotherapies available for treating patients with lung cancer. The selection of these targeted chemotherapies is based on the presence of three somatic mutations: L858R and Del 747,_A750 for Afatinib, Gefitinib, or erlotinib and T790M for osimertinib. There is a number of methods including FDA approved methods for the detection of L858R and Del 747,_A750 for and T790M mutations from FFPE samples.

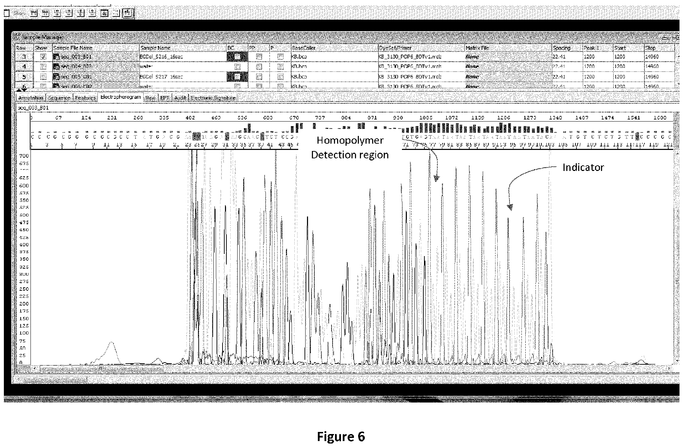

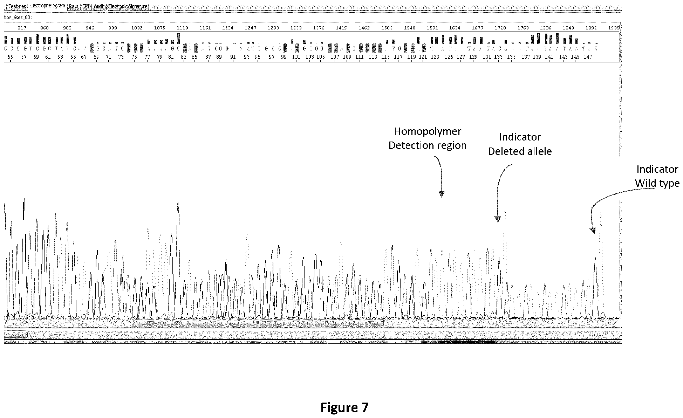

Each PCR reaction included 25.0 µl of 2X buffer (MultiPlex PCR Master Mix, Qiagen). 1.0 µl each of the 10 pmol forward and reverse primers were added to the reaction with 1.0 µl of the corresponding control clone. Appropriate amount of water was added to bring the final volume to 50.0 µl. PCR conditions: 95C/5min, (95C/30sec, 57.5C/90sec, 72C/30sec) x35, 68C/10min. Following PCR clean up sequencing reaction was set up using 1.0 µl of Big dye, 9.5 µl of 5X sequencing buffer, 1 pmoles of sequencing primer and 30 µl of the purified PCR products and 4.5 µl of Dnase free water to a total reaction volume of 50 µl. Cycle Sequencing conditions: (96C/105sec, 55C/10sec, 60C/2.5min) x25. Cycle sequencing products were cleaned with CleanSEQ (Beckman Agencourt USA) and eluted in 40 µl of Dnase free water. The cleaned products injected for 16 sec, to Genetic analyzer 3130xl and the electropherogram was analyzed using sequencing analysis software 6.0. The electropherogram shows wild type with EGFR deletion (see Fig. 6 and 7).

### EGFR PCR primers

Upper primer 5'- GTG TGA TTC GTG GAG CCC AAC AG-3'
Lower primer 5'- GTATTATTATTATTATTATTATTATTACTC ACA TCG AGG ATT TCC TTG TTG GC

Sequencing primer:
Mutant 5' CTC ACA TCG AGG ATT TCC TTG TTG GC-3'
Wild type: 5' TTTTTTTTTTTTTTAAAGTTAAAATTCCCGTCGCTATCAAGG-3'

### Example 2: Determination of Microsatellite variants.

Determination of micro satellite variants that are associated with human diseases. Hereditary nonpolyposis colorectal cancer (HNPCC) commonly referred as Lynch syndrome is an autosomal hereditary condition that is associated with a number of cancers including colorectal cancer. The major steps are: PCR primer construction. The PCR primers are designed so that the lower primer has a repeat adenosine nucleotide with guanidine as the last nucleotide. The target segment is amplified using an upper primer and the modified lower. PCR products are cleaned and sequenced using a sequencing primer upstream of the region of microsatellite variant. The cycle sequencing mixture is purified and analyzed in a capillary electrophoresis.

Each PCR reaction included 25.0 µl of 2X buffer (MultiPlex PCR Master Mix, Qiagen). 1.0 µl each of the 10 pmol forward and reverse primers were added to the reaction with 1.0 µl of the corresponding control clone. Appropriate amount of water was added to bring the final volume to 50.0 1. PCR conditions: 95C/5min, (95C/30sec, 57.5C/90sec, 72C/30sec) x35, 68C/10min. Following PCR clean up sequencing reaction was set up using 1.0 µl of Big dye, 9.5 µl of 5X sequencing buffer, 1pmoles of sequencing primer and 30 µl of the purified PCR products and 4.5 µl of Dnase free water to a total reaction volume of 50 µl. Cycle Sequencing conditions: (96C/105sec, 55C/10sec, 60C/2.5min) x25. Cycle sequencing products were cleaned with CleanSEQ (Beckman Agencourt USA) and eluted in 40 µl of Dnase free water. The cleaned products injected for 16 sec to Genetic analyzer 3130xl and the electropherogram was analyzed using sequencing analysis software 6.0. The electropherogram shows the wild type indicator as well as the deleted region (see Fig. 6 and 7).

### Example 3: Determination of Microbial speciation

### Disease condition: Vaginosis. Candida speciation such as C. albicans, C. glabrata, C.kruesi, C.tropicalis, C.parapsilosis

Each PCR reaction included 25.0 µl of 2X buffer (MultiPlex PCR Master Mix, Qiagen). 1.0 µl each of the 10 pmol forward and reverse primers were added to the reaction with 1.0 µl of the corresponding control clone. Appropriate amount of water was added to bring the final volume to 50.0 µl. PCR conditions: 95C/5min, (95C/30sec, 57.5C/90sec, 72C/30sec) x35, 68C/10min. Following PCR clean up sequencing reaction was set up using 1.0 µl of Big dye, 9.5 µl of 5X sequencing buffer, 1 pmoles of sequencing primer and 30 µl of the purified PCR products and 4.5 ml of Dnase free water to a total reaction volume of 50 µl. Cycle Sequencing conditions: (96C/105sec, 55C/10sec, 60C/2.5min) x25. Cycle sequencing products were cleaned with CleanSEQ (Beckman Agencourt USA) and eluted in 40 µl of Dnase free water. The cleaned products injected for 16 sec to Genetic analyzer 3130xl and the electropherogram was analyzed using sequencing analysis software 6.0.

Concept of primers:

| | Sequencing primer | Species seq | Lower primer | Read seq |
|---|---|---|---|---|
| Candida albicans: | TTTTTCGATGCGTACTGG | ACCAG | CCGAGCCTTTCCTTC | TTAAC |
| Candida glabrata: | TTGGCCGGTCCATCTTTC | TGATG | CGTACTGGACCCAAC | AATTG |
| Candida tropicalis: | TCAATATGGGTAGGAAAA | GTGAC | CCTACCATGGTTTCC | CCGGT |
| Candida parasilosis: | GCCCCGTGGGATGCCGGC | GGAAG | CAGTGAGGCCCTTCT | TTTTA |

### Reference cited

### US PATENT DOCUMENTS

| | | |
|---|---|---|
| 1. | 9,005,425 | Burrows et al |
| 2. | 8.771,965 | Sarkar et al |
| 3. | 8,754,191 | Nielsen et al |
| 4. | 8,828,391 | Denis, et al- EGFR |
| 5. | 8,788,215 | Chakraborty et al- STR |

### OTHER PUBLICATIONS

1. Marie B., et al. Assessment of EGFR Mutation Status in Lung Adenocarcinoma by Immunohistochemistry Using Antibodies Specific to the Two Major Forms of Mutant EGFR. Journal of Molecular Diagnostics, Vol. 12, No. 2, March 2010.
2. Pagin,A, F Zerimech, J Leclerc, A Wacrenier, S Lejeune, C Descarpentries, F Escande, N Porchet, M-P Buisine. Evaluation of a new panel of six mononucleotide repeat markers for the detection of DNA mismatch repair-deficient tumors. British Journal of Cancer volume 108, pages 2079-2087 28 May 2013.
3. G Perinchery, D Nojima, R Goharderakhshan, Y Tanaka, J Alonzo, R Dahiya Microsatellite instability of dinucleotide tandem repeat sequences is higher than trinucleotide, tetranucleotide and pentanucleotide repeat sequences in prostate cancer. International Journal of Oncology. June 1, 2000, Pages:1203-1212 https://doi.org/10.3892/ijo.16.6.1203.
4. Oliver J, et al., Reduced penetrance alleles for Huntington's disease: a multi-center direct observational study. J Med Genet. 2007 Mar; 44(3): e68.
5. Chehap et al., A dimorphic 4-bp repeat in the cystic fibrosis gene is in absolute linkage disequilibrium with the delta F508 mutation: implications for prenatal diagnosis and mutation origin. Am J Hum Genet. 1991 Feb; 48(2): 223-226.
6. Tsukino H et al.,Effects of cytochrome P450 (CYP) 2A6 gene deletion and CYP2E1 genotypes on gastric adenocarcinoma. Int J Cancer. 2002 Aug 1;100(4):425-8.
7. Sartor O, Dong Y. Androgen receptor variant-7: an important predictive biomarker in castrate resistant prostate cancer. Asian J Androl. 2015 May-Jun;17(3):439-40. doi: 10.4103/1008-682X.145069.
8. McNamara-Schroeder K1, Olonan C, Chu S, Montoya MC, Alviri M, Ginty S, Love J.J. DNA fingerprint analysis of three short tandem repeat (STR) loci for biochemistry and forensic science laboratory courses. Biochem. Mol. Biol. Educ. 2006 Sep;34(5):378-83. doi: 10.1002/bmb.2006.494034052665.
9. Norikazu Matsuo . Association of EGFR Exon 19 Deletion and EGFR-TKI Treatment Duration with Frequency of T790M Mutation in EGFR-Mutant Lung Cancer Patients. Sci Rep. 2016; 6: 36458.
10. Kosaka, T et al., Response heterogeneity of EGFR and HER2 exon 20 insertions to covalent EGFR and HER2 inhibitors. Cancer Res. 2017 May 15; 77(10): 2712-2721.
11. Reungwetwattana.T and Sai-Hong Ignatius Ou MET exon 14 deletion (METex14): finally, a frequent-enough actionable oncogenic driver mutation in non-small cell lung cancer to lead MET inhibitors out of "40 years of wilderness" and into a clear path of regulatory approval. Transl Lung Cancer Res. 2015 Dec; 4(6): 820-824.
12. Shaw, A.T and Jeffrey A. Engelman. ALK in Lung Cancer: Past, Present, and Future. J Clin Oncol. 2013 Mar 10; 31(8): 1105-1111.
13. Bubendorf, L et al., Testing for ROS1 in non-small cell lung cancer: a review with recommendations. Virchows Arch. 2016; 469(5): 489-503.
14. Snehal Dabir et al., RET Mutation and Expression in Small-Cell Lung Cancer. Journal of Thoracic Oncology. Volume 9, Issue 9, September 2014, Pages 1316-1323
15. Su Jin Lee et al., NTRK gene amplification in patients with metastatic cancer. Precision and Future Medicine 2017; 1(3): 129-137.
16. Sardi T.C.O et al., Candida species: current epidemiology, pathogenicity, biofilm formation, natural antifungal products and new therapeutic options. Journal of Medical Microbiology (2013), 62, 10-24.
17. Li K, Yin R, Wang D, Li Q. Human papillomavirus subtypes distribution among 2309 cervical cancer patients in West China. Oncotarget. 2017 Apr 25;8(17):28502-28509. doi: 10.18632/oncotarget. 16093.

## Claims

1. A method of detecting one or more deoxyribose nucleotide (DNA) template(s) comprising the steps of:
- amplifying segments of a DNA template by polymerase chain reaction using upper primers and lower primers, wherein each of the lower primers comprises a homopolymer region at its 5' end comprising a homopolymer detection and an indicator region, and repeated nucleotides, wherein the number of repeated nucleotides in the lower primers are identical or differ from each other, and
- generating a nucleotide sequence consisting of a nucleotide sequence that is complimentary to the lower primer, the detection region and the indicator region using a sequencing primer annealing within an amplicon, and
- aligning the generated nucleotide sequence with a reference sequence to determine the location of the indicator nucleotide in the reference nucleotide sequence and in the DNA template and thereby identifying the DNA template.

2. The method of claim 1, wherein the DNA template comprises a genetic variant comprising a deletion region compared to the corresponding wild type DNA template, and the upper primers and lower primers amplify a segment of the DNA template encompassing the deletion, where the upper primers anneal upstream of the deletion region and the lower primers anneal downstream of the deletion region, and the sequencing primer anneals within the amplicon upstream of the deletion generating a nucleotide sequence that corresponds to a nucleotide sequence downstream of the deletion region, and the generated nucleotide sequence is complimentary to the lower primer, the homopolymer detection region and the indicator region at the 5' end.

3. The method of claim 1, wherein the DNA template comprises a genetic variant comprising an insertion region compared to the corresponding wild type DNA template, and the upper primers and the lower primers amplify a segment of the DNA template encompassing the insertion region, where the upper primers anneal upstream of the insertion region and the lower primers anneal downstream of insertion region, and the sequencing primer anneals within the amplicon upstream of the insertion region generating a nucleotide sequence that corresponds to a nucleotide sequence down-stream of the insertion region, and the generated nucleotide sequence is complimentary to the lower primer, the homopolymer detection region and the indicator region at the 5' end.

4. The method of claim 1, wherein the DNA templates comprises a genetic variant comprising a substitution of one or more nucleotide sequences of deoxyribose nucleotide.

5. The method of any one of claims 1-4, wherein the upper primer comprises a homopolymer region at its 5' end comprising a detection and an indicator region.

6. The method of any one of claims 1-4, wherein both the upper primer and the lower primer comprise the homopolymer region at the 5' end comprising a detection and an indicator region.

7. The method of any one of claims 1-6 wherein the DNA template comprises a ribose nucleotide.

8. The method of any one of claims 1-7, wherein the amplification is by ligase chain reaction.

9. The method of any one of claims 2, 5-7, wherein a part of the upper primer anneals upstream of the deletion region and the other part of the upper primer anneals downstream of the deletion region.

10. The method of any one of claims 2, 5-7, wherein a part of the sequencing primer anneals upstream of the deletion region and the other part of the upper primer anneals downstream of the deletion region.

11. The method of any one of claims 3, 5-7, where a part of the upper primer anneals in the insertion region and the other part anneals downstream of the insertion region.

12. The method of any one of claims 3, 5-7, where the upper primer anneals completely in the insertion region.

13. The method of any one of claims 3, 5-7, where a part of the sequencing primer anneals in the insertion region and the other part anneals downstream of the insertion region.

14. The method of any one of claims 3, 5-7, where the sequencing primer anneals completely in the insertion region.

15. The method of any one of claims 4, 5-7, where the method comprises at least two sequencing primers, one binding specifically to a mutated allele comprising a mutant nucleotide at its 3' end and annealing within the region of the amplicon generating a nucleotide sequence that consists of a nucleotide sequence downstream of a nucleotide substitution locus, wherein the nucleotide sequence is complimentary to the lower primer, and the detection and indicator region, and
that the other is specifically binding the wild type allele comprising the wildtype nucleotide at its 3' end, anneal within the region of the amplicon generating a nucleotide sequence that consists of a nucleotide sequence downstream of the nucleotide substitution locus, wherein the nucleotide sequence is complimentary to the lower primer, and the detection and indicator region and,
wherein the generated nucleotide sequence is aligned with the nucleotide sequence of the wild type, and the difference of the location of the marker nucleotide between the wild type nucleotide sequence and the generated nucleotide sequence determines the presence of the deletion in the DNA template.

16. The method of claim 1, wherein a fusion DNA template comprises an insertion region, and the upper primers and the lower primers amplify a segment of the DNA template encompassing the fusion region, where the upper primers anneal upstream of the region junction of the fusion and the lower primers anneal down- stream of the junction of the fusion region, and
the sequencing primer anneals within the amplicon upstream of the junction of the fusion region generating a nucleotide sequence that corresponds to a nucleotide sequence down-stream of the junction of the fusion region, and the generated nucleotide sequence is complimentary to the lower primer, the homopolymer detection region and the indicator region at the 5' end.

17. The method of claim 1, wherein the DNA template comprises different genomes, and the upper primers and lower primers amplify a segment of the DNA template, the genome specific sequencing primer anneals within the amplicon generating a nucleotide sequence complimentary to the lower primer, the homopolymer detection region and the indicator region at the 5' end and the location of the indicator region identifies its corresponding genome.

18. Kit for detecting a DNA template based on a method according to one of claims 1 to 17, comprising one or more upper or lower primer(s) or a mixture thereof, a sequencing primer and optionally a reference sequence.
